# EUROPÄISCHE PATENTSCHRIFT

(11) **EP 1 593 952 B1**
(45) Veröffentlichungstag und Bekanntmachung des Hinweises auf die Patenterteilung: **15.04.2009**
(21) Anmeldenummer: 04010717.9
(22) Anmeldetag: 05.05.2004
(51) Int. Cl.: G01N 1/44, G01N 1/40

(54) **Vorrichtung und Verfahren zur diskontinuierlichen Hydrolyse**
Apparatus and method for discontinuous hydrolysis
Dispositif et méthode d'hydrolyse discontinue

(43) Veröffentlichungstag der Anmeldung: 09.11.2005
(73) Patentinhaber: C. Gerhardt Fabrik und Lager chemischer Apparate GmbH & Co. KG, 53639 Koenigswinter (DE)
(72) Erfinder: Jeub, Uwe, 53913 Swisttal (DE); Kranz, Markus, 53562 Sankt Katharinen (DE); Kurth, Heinz-Jürgen, 52525 Heinsberg (DE)
(74) Vertreter: Grünecker, Kinkeldey, Stockmair & Schwanhäusser Anwaltssozietät

(56) Entgegenhaltungen:
- WO-A1-97/44109
- WO-A2-02/45812
- GB-A- 1 187 272
- US-B1- 6 479 295

## Beschreibung

Die vorliegende Erfindung betrifft ein Verfahren und eine Vorrichtung zur diskontinuierlichen Hydrolyse von Laborproben sowie ein Verfahren zur diskontinuierlichen Hydrolyse einer Laborprobe. Ferner betrifft die vorliegende Erfindung einen Probenbehälter für die Hydrolysevorrichtung nach der vorliegenden Erfindung.

Insbesondere in der Labortechnik wird heutzutage häufig die Hydrolyse eingesetzt, um mittels Lösungsmittelextraktion vor der Gesamtfettbestimmung, beispielsweise von Nahrungs- und Futtermittelproben, einen Säureaufschluss zu erzielen. Im Labormaßstab werden hierzu üblicherweise Proben von bis zu 5 g in 100 ml einer 15%igen Salzsäure für etwa 1 Stunde gekocht. Dann wird das Lösungsmittel auf einen Filter abgefiltert. Danach wird die Probe mit bis zu 800 ml warmem destilliertem Wasser gespült, bis diese säurefrei ist.

Die Hydrolyse stellt sich in der heutigen Labortechnik als lästiger, zeitaufwendiger Vorgang dar, dessen Durchführung sehr viel Handarbeit erfordert. Insbesondere das Abfiltern und Auswaschen der Probe ist mühsam, da das Waschwasser nur in kleinen Portionen zugeführt werden kann und ein die Probe aufnehmendes Kochgefäß sorgfältig von Probenresten frei gewaschen werden muss.

Der vorliegenden Erfindung liegt das Problem zugrunde, eine Hydrolysevorrichtung zur diskontinuierlichen Hydrolyse von Laborproben anzugeben, die eine leichtere Durchführung der Hydrolyse ermöglicht. Ferner soll mit der vorliegenden Erfindung ein vereinfachtes Verfahren zur Hydrolyse einer Laborprobe angegeben werden.

Im Hinblick auf das vorrichtungsmäßige Problem wird mit der vorliegenden Erfindung eine Hydrolysevorrichtung mit den Merkmalen von Anspruch 1 angegeben. Diese weist einen verschließbaren Probenbehälter auf, der mit drei Anschlüssen versehen ist, nämlich einem Säure-, einem Spülwasser- und einem Gasanschluss und an dem ein bis zu dem Behälterboden reichendes Siphon angeordnet ist. Mit Rücksicht auf die Verschließbarkeit weist der Behälter beispielsweise einen Deckel auf, der verschwenkbar an einem die Probe aufnehmenden Behälterteil, beispielsweise einem Probenglas angeordnet ist. Der Probenbehälter ist so weit verschließbar, dass jedenfalls aufgrund eines über den Gasanschluss in den Behälterinnenraum einströmenden Gases ein Überdruck in dem Behälter erzielt werden kann. Vermöge dieses Innendrucks wird eine in dem Behälter befindliche Flüssigkeit über den Siphon aus dem Behälter herausgedrückt. Der Siphon weist hierzu ein unteres Ende auf, welches sich im Bereich des Behälterbodens befindet; im Hinblick auf eine möglichst vollständige Entleerung des Behälters durch den Siphon ist vorzugsweise im Bereich des Behälterbodens eine Senke vorgesehen, in der das behälterseitige Ende des Siphons angeordnet ist. Durch den Säureanschluss kann zunächst die für den Säureaufschluss erforderliche Säure in den Behälter eingeleitet werden; durch den Spülwasseranschluss das zum Reinigen der Probe und/oder des Behälters erforderliche Spülwasser.

Die erfindungsgemäße Hydrolyseeinrichtung erlaubt die vollautomatische Durchführung der hydrolyseerforderlichen Schritte. Die erfindungsgemäße Hydrolysevorrichtung entlastet den Benutzer davon, händig Substanzen zur Durchführung der Hydrolyse in das Kochgefäß einzubringen. Da ferner die in dem Probenbehälter enthaltenen Substanzen durch Aufbringen eines Überdrucks ausgefördert werden, muss der Probenbehälter weder durch Verschwenken noch durch Handbetätigen eines Ventils entleert werden. Die Handhabung der erfindungsgemäßen Hydrolysevorrichtung ist dementsprechend einfach und erlaubt eine sichere Durchführung der Hydrolyse, ohne dass die Gefahr besteht, dass der Benutzer mit den mitunter aggressiven Substanzen in Berührung kommt.

Der Siphon kann an dem Boden des Probenbehälters angeordnet sein und von dort sich außerhalb des Behälters bis über den gewünschten Füllstand erstrecken oder aber einen bis zu dem Behälterboden reichenden und in dem Behälter angeordneten Siphonabschnitt aufweisen, der oberhalb des gewünschten Füllstandes des Probenbehälters bei der Durchführung der Hydrolyse durch die Seitenwandung des Probenbehälters hindurchgeführt ist. Im Hinblick auf ein durch Überdruck einzuleitendes Abführen einzelner flüssiger Substanzen aus dem Probenbehälter ist es allein von Bedeutung, dass der oberste Punkt des Siphons oberhalb des gewünschten Füllstandes des Probenbehälters liegt.

Eine besonders einfache Ausgestaltung wird gemäß einer bevorzugten Weiterbildung der Erfindung dadurch angegeben, dass jedenfalls der Säure-, der Spülmittel- und der Gasanschluss an einem Deckel des Probenbehälters angeordnet sind. Dies lässt die Möglichkeit offen, den die Probe aufnehmenden Teil des Probenbehälters, der nachfolgend als Probenglas bezeichnet wird, relativ einfach auszubilden, beispielsweise durch ein zylindrisches Glas. Diese Möglichkeit ist insbesondere dann gegeben, wenn auch der Siphon an dem Deckel befestigt ist. Der Deckel sollte vorzugsweise derart bemessen sein, dass die von dem Deckel abgedichtete Öffnung ein Einlegen und Entnehmen der Probe in dem Probenbehälter ermöglicht.

Gemäß einer weiteren bevorzugten Ausgestaltung, bei welcher der Deckel vorzugsweise ein das Probenglas oberseitig verschließender Deckel ist, weist dieser einen mit dem Behälter kommunizierenden Kondensator auf, durch den insbesondere beim Kochen der Probe aufsteigende Dämpfe kondensiert und in den flüssigen Zustand zurückgeführt werden.

Weiterhin und ebenfalls zur Vereinfachung der konstruktiven Ausgestaltung der erfindungsgemäßen Hydrolysevorrichtung ist der Kondensator vorzugsweise an dem Deckel befestigt und kommuniziert über eine Kondensatoröffnung mit dem Inneren des Behälters. Auf der dem Behälter gegenüberliegenden, vorzugsweise oberhalb des Behälters angeordneten Seite, weist der Kondensator eine Kondensatorsäule auf, die eine Kühlmittelleitung umgibt.

Im Hinblick auf eine möglichst flächige Verteilung des Spülmittels in dem Behälter mündet der Spülmittelanschluss behälterseitig vorzugsweise in einem Sprühkopf. Dieser ist insbesondere an dem Deckel, hier mittig an dem Deckel befestigt, um das eingebrachte Spülmittel beispielsweise auch gegen den Deckel und die Seitenwandungen des Behälters zu spritzen.

Dem Behälterboden ist vorzugsweise eine Heizeinrichtung zugeordnet, womit gemeint ist, dass die Heizeinrichtung zwar in vorbestimmter Lage in Bezug auf den Probenbehälter angeordnet werden kann, jedoch nicht zwingend fester Bestandteil des Behälters ist. So kann die Heizeinrichtung, insbesondere im Hinblick auf einen Austausch des Probenglases, beispielsweise bei einer Zerstörung desselben, lösbar im Bereich des Behälterbodens befestigt sein und/oder Teil einer Probenbehälterhalterung sein, auf der sich der Probenbehälter abstützt.

Die Hydrolysevorrichtung weist vorzugsweise ein an dem anderen Ende des Siphon angeschlossenes Ventil auf, das von einem die Füllhöhe einer nachgeordneten Filtriereinrichtung erfassenden Sensor gesteuert wird. Diese bevorzugte Weiterbildung erlaubt ein kontrolliertes Ableiten einer Substanz aus dem Probenbehälter, ohne dass ein konstanter Volumenstrom bestimmt und gesteuert und/oder eine volumetrische Messung des Volumenstromes durchgeführt werden muss. Das an dem anderen Ende des Siphons angeschlossene Ventil verlegt lediglich den Ausgang der Substanz aus dem Behälter, wohingegen der die Substanz aus dem Probenbehälter fördernde Gradient, d.h. der Überdruck, im Hinblick auf eine Steuerung der Volumenmenge unverändert bleiben kann. Das Ventil wird bei dieser bevorzugten Ausgestaltung von einem Sensor gesteuert, der die Füllhöhe einer nachgeordneten Filtriereinrichtung erfasst, so dass die Steuerung letztlich im Hinblick auf den Durchsatz des nachgeordneten Verfahrensschritts, nämlich das Filtrieren der abgeleiteten Substanz erfolgen kann.

Wiederum im Hinblick auf eine leichte Handhabung, insbesondere Reinigung der erfindungsgemäßen Hydrolysevorrichtung wird gemäß einer weiteren bevorzugten Ausgestaltung der vorliegenden Erfindung vorgeschlagen, dass der Sensor ein auf den Flüssigkeitsspiegel der Filtriereinrichtung gerichteter und durch Reflektion die Füllhöhe derselben ermittelnder Sensor ist. Die Steuerung der Durchflussmessung erfolgt dementsprechend berührungsfrei, so dass der hier zum Einsatz kommende Sensor weder durch die in der Hydrolysevorrichtung zum Einsatz kommenden Substanzen, noch durch eine nachfolgende Reinigung der Hydrolysevorrichtung in Mitleidenschaft gezogen werden kann. Im Hinblick auf einen vollautomatischen Betrieb der Hydrolysevorrichtung wird gemäß einer weiteren bevorzugten Ausgestaltung der vorliegenden Erfindung vorgeschlagen, eine Steuereinheit vorzusehen, die eine Säuredosiereinrichtung, die mit dem Säureanschluss verbunden ist, ein Druckventil, welches mit dem Gasanschluss verbunden ist, sowie eine Spülwasserdosiereinrichtung steuert, die mit dem Spülmittelanschluss verbunden ist. Die Steuereinheit übernimmt die Abfolge der verschiedenen Phasen der Hydrolyse, bei welcher in einer ersten Phase zunächst die Säure in den Probenbehälter eingeleitet, danach in der zweiten Phase durch Aufgeben von Druckluft durch den Siphon ausgefördert und schließlich in einer dritten Phase in ein oder mehreren Spülvorgängen Spülwasser in den Probenbehälter eingeleitet und durch Beaufschlagen mit Druckluft aus diesem über den Siphon ausgefördert wird. Die Säuredosiereinrichtung wirkt mit einem Säurereservoir zusammen und fördert in diesem Säurereservoir enthaltene Säure zu dem Säureanschluss und von dort in den Probenbehälter. Das Druckventil kann beispielsweise in einer druckbeaufschlagten Leitung zwischen einer Luftdruckquelle und dem Gasanschluss zwischengeschaltet sein. Vorzugsweise greift die Spülwasserdosiereinrichtung auf ein Spülreservoir zu, in dem destilliertes Wasser gehalten ist und welches vorzugsweise eine Heizung aufweist, um dieses destillierte und in dem Reservoir aufgenommene Wasser auf einer vorbestimmten Temperatur zu halten.

Gemäß einer weiteren bevorzugten Ausgestaltung, die im Hinblick auf eine vollständige Automatisierung vorgeschlagen wird, wirkt die vorerwähnte Steuereinheit ferner mit einer Benetzungseinrichtung zusammen, welche Benetzungsflüssigkeit, beispielsweise destilliertes Wasser auf die Filtriereinrichtung aufgibt, um insbesondere einen in der Filtriereinrichtung aufgenommenen Filter vor der ersten Filtration, d.h. vor dem Ausförderung von Säure aus dem Probenbehälter zu benetzen.

Im Hinblick auf das verfahrensmäßige Problem wird mit der vorliegenden Erfindung ein Verfahren zur diskontinuierlichen Hydrolyse einer Laborprobe vorgeschlagen, bei dem in an sich bekannter Weise eine Probe in einen Probenbehälter eingebracht, in Säure gekocht und nachfolgend gespült wird. Im Hinblick auf eine Vereinfachung des erfindungsgemäßen Verfahrens wird die Probe in einen luftdicht verschließbaren Probenbehälter eingebracht, der nachfolgend verschlossen wird. Danach wird ein automatisiertes Hydrolyseprogramm gestartet, welches eine Säuredosiereinrichtung derart steuert, dass eine vorbestimmte Säuremenge in dem Probenbehälter eingebracht wird, danach eine dem Probenbehälter zugeordnete Heizeinrichtung derart steuert, dass die in dem Probenbehälter befindliche Säure über eine vorbestimmte Kochdauer auf einer vorbestimmten Temperatur gehalten wird. Sodann steuert das Hydrolyseprogramm ein dem Probebehälter zugeordnetes Druckventil, um den Innendruck in dem Probenbehälter soweit zu erhöhen, dass die Säure durch einen Siphon des Probenbehälters aufsteigt und aus diesem abfließt und in einer nachgeordneten Filtriereinrichtung gefiltert wird. Der dichte Verschluss des Probenbehälters hat insbesondere im Hinblick auf diesen Verfahrensschritt zu erfolgen, bei dem der Probenbehälter jedenfalls so luftdicht verschlossen sein muss, dass sich ein hinreichender Innendruck in dem Behälter aufbaut, dass die Säure über einen oberen Krümmungspunkt des Siphons aus dem Probenbehälter abgefördert werden kann. Danach steuert das Hydrolyseprogramm eine mit dem Probenbehälter kommunizierende Spülmitteldosiereinrichtung, um ein vorbestimmbares Volumen an Spülflüssigkeit in den Probenbehälter einzubringen. Schließlich wird nach Ablauf einer vorbestimmbaren Spüldauer von dem Hydrolyseprogramm erneut das Druckventil angesteuert, um den Innendruck in dem Probenbehälter soweit zu erhöhen, dass die Spülflüssigkeit durch den Siphon aufsteigt und ebenfalls gefiltert wird. Bei dem erfindungsgemäßen Verfahren kann das Hydrolyseverfahren in sämtlichen Phasen aufgrund der Steuerung des automatisiert ablaufenden Hydrolyseprogramms ohne Eingriff eines Benutzers durchgeführt werden. Das erfindungsgemäße Verfahren wird dadurch erheblich vereinfacht und die Störanfälligkeit des Verfahrens sowie die Akzeptanz des Hydrolyseverfahrens für kleinere Laborproben erhöht.

Bevorzugte Weiterbildungen des erfindungsgemäßen Verfahrens sind in den Ansprüchen 14 bis 17 angegeben.

Die vorliegende Erfindung wird nachfolgend anhand eines Ausführungsbeispiels in Verbindung mit der Zeichnung näher erläutert. In dieser zeigen:
- Figur 1: ein Ausführungsbeispiel einer Hydrolysevorrichtung mit einer in dieser eingelegten Probe; und

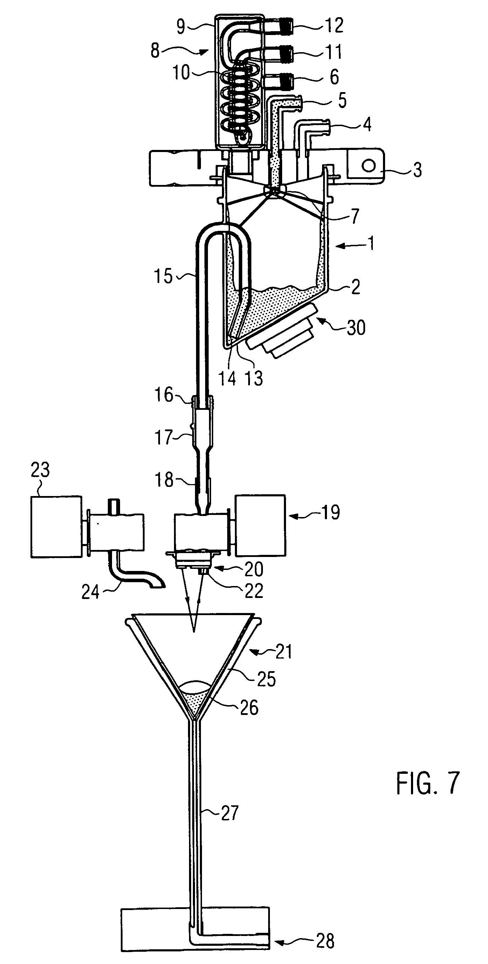

- Figuren 2-9: das in Figur 1 gezeigte Ausführungsbeispiel in mehreren Phasen der Hydrolyse.

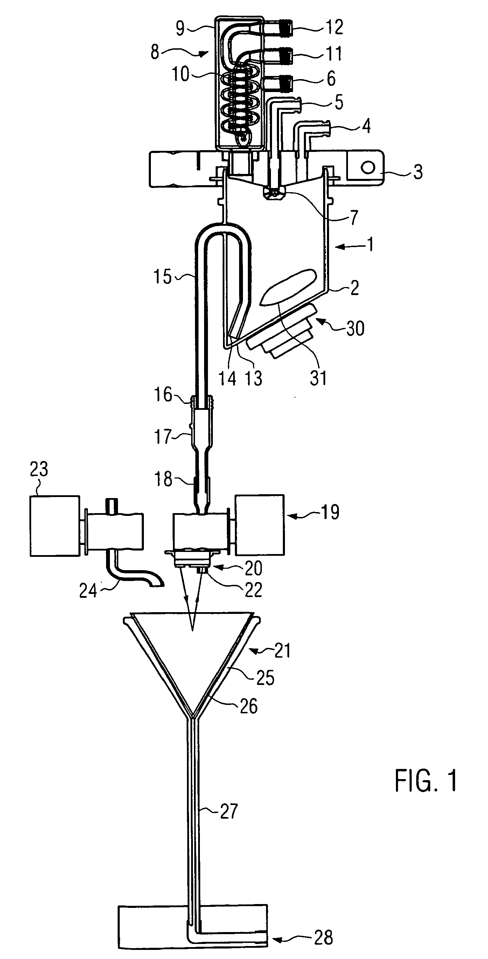

Das in Figur 1 gezeigte Ausführungsbeispiel einer Hydrolyseeinrichtung umfasst als zentrales Element derselben einen Probenbehälter 1 mit einem Probenglas 2 und einem luftdicht auf das Probenglas 2 aufsetzbarem Deckel 3. Bei dem gezeigten Ausführungsbeispiel weist der Deckel 3 hierfür eine hier nicht näher dargestellte Probenglasfassung auf, in welche das Probenglas 2 dichtend eingesetzt ist und welche ein verschwenkbar daran befestigtes Deckeloberteil trägt. Das Probenglas 2 kann aus geblasenem Glas bestehen und durch an das Probenglas 2 angepasste Dicht- und Halteflächen dichtend an dem Deckel 3 gehalten werden.

Der Deckel 3 trägt den Deckel 3 oberseitig überragende Anschlüsse für Säure (Bezugszeichen 4) für Spülwasser (Bezugszeichen 5) und für Druckluft (Bezugszeichen 6). Die Anschlüsse 4 und 5 für Säure bzw. Spülwasser sind in Form eines U-förmigen Rohrstücks ausgebildet und münden unmittelbar in den von dem Probenglas 2 ausgesparten Probenaufnahmeraum. An dem behälterseitigen Ende des Spülwasseranschlusses 5 befindet sich ein Duschkopf 7, der im Wesentlichen auf die Wandung des Probenglases 2 und die gehäuseseitige Innenwand des Deckels 3 gerichtete Strömungskanäle aufweist, die mit dem Spülwasseranschluss 5 kommunizieren. Der Druckluftanschluss 6 ist an einen Kondensator 8 angeschlossen, welche eine an den Deckel 3 angeschlossene Kondensatorsäule 9 umfasst, die eine Kühlmittelleitung 10 umgibt, deren Zu- und Ablaufanschlüsse für Kühlwasser mit Bezugszeichen 11 und 12 gekennzeichnet sind.

Das Probenglas 2 hat einen schräg geneigten Boden 13, der mit einer Seitenwand des Probenglases 2 eine Senke des Bodens 13 ausbildet. In dieser Senke endet ein behälterseitiges Ende 14 eines Siphons 15, dessen oberer Scheitelpunkt durch die Wandung des Probenglases 2 hindurchgeführt ist. Das andere Ende 16 des Siphons 15 mündet in ein Adapterstück 17, welches an seinem anderen Ende mit einem Einlassstutzen 18 eines Ventils 19 verbunden ist.

Auf der Außenseite des Probenglases 2 befindet sich eine mit dem Probenglasboden 13 in Berührung stehende und schematisch dargestellte Heizung 30, die mittels Wärmeleitung durch den Probenboden 13 auf den Innenraum des Probenbehälters 1 wirkt. Über der Heizung 30 ist eine schematisch dargestellte Laborprobe 31 zu erkennen.

Das Gehäuse des Ventils 19 trägt eine Füllstands-Sensoreinheit 20, die ein optisches Signal in Richtung auf einen Filter 21 ausgibt, welches auf der Flüssigkeitsoberfläche des Filters 21 reflektiert und von einem Sensor 22 der Sensoreinheit 20 erfasst und als Maß für den Füllstand des Filters 21 ausgewertet wird.

Im Wesentlichen auf gleicher Höhe mit der Füllstands-Sensoreinheit 20 befindet sich oberhalb des Filters 21 eine Benetzungseinrichtung 23, die mit einem Reservoir für destilliertes Wasser verbunden ist und ein oberhalb des Filters 21 endendes Rohrstück 24 umfasst, welches mit dem Reservoir für destilliertes Wasser zumindest bei Betätigung der Benetzungseinrichtung 23 kommuniziert und destilliertes Wasser auf den Filter 21 aufgibt.

Dieser Filter 21 umfasst einen Filtertrichter 25, in dem ein Filterpapier 26 eingelegt ist und der mit einem Ablaufrohr 27 kommuniziert, welches in einen Ablauf 28 mündet.

Die Benetzungseinrichtung 23, sowie eine hier nicht dargestellte und dem Drucklufteinlass 6 zugeordnete Druckluftquelle, eine mit dem Spülwasseranschluss 5 kommunizierende Spülwasserdosiereinrichtung sowie eine mit dem Säureanschluss 4 kommunizierende Säuredosiereinrichtung sind mit einer hier nicht dargestellten Steuereinheit verbunden. Diese steuert vorzugsweise auch die Heizung 30 und gegebenenfalls die Füllstands-Sensoreinheit 20.

### Das in Figur 1 gezeigte Ausführungsbeispiel wird wie folgt betrieben:

Zunächst wird der Deckel 3 gegenüber dem Probenglas 2 verschwenkt und der Probenbehälter geöffnet. Die Laborprobe 31 wird in dem Probenbehälter 1 eingelegt. Danach wird der Probenbehälter 1 im Wesentlichen gasdicht verschlossen. Da auch das Ventil 19 geschlossen ist, befindet sich die Probe 31 in einem abgeschlossenen Raum.

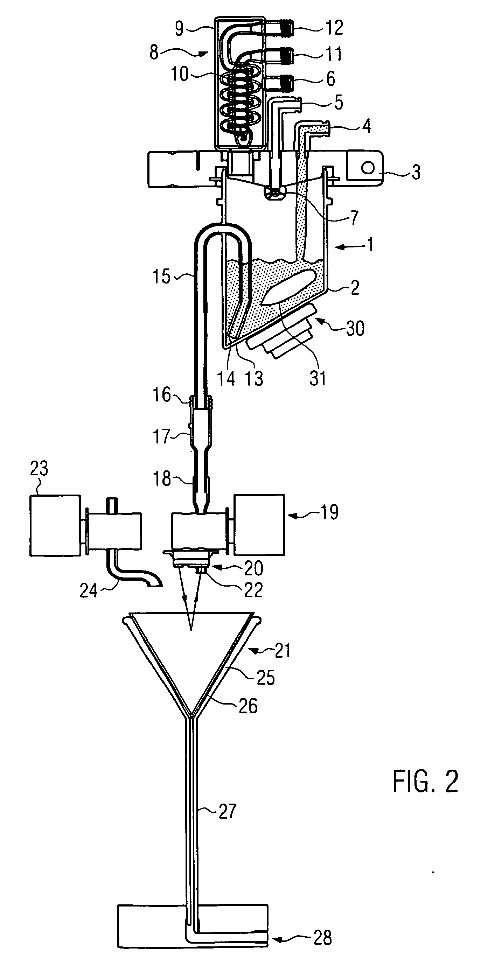

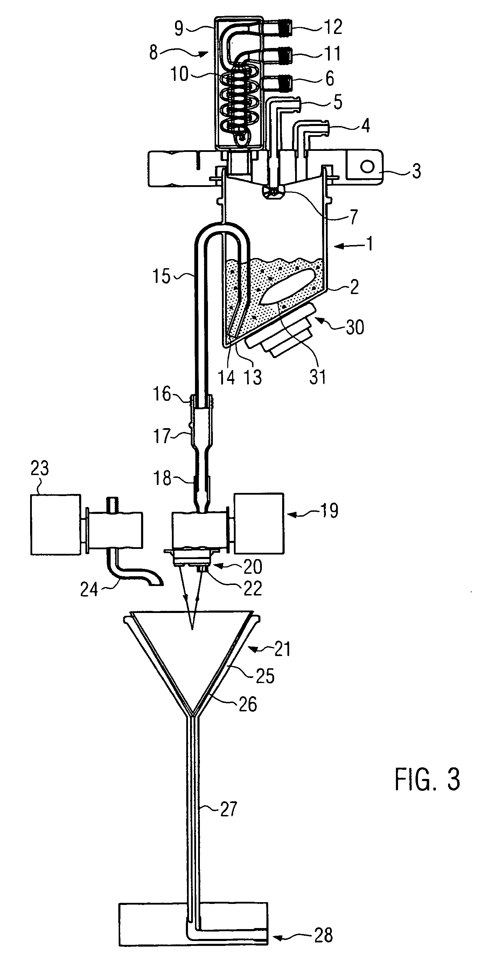

Nun wird die Aufschlussphase eingeleitet, in der die Steuereinheit die Säuredosiereinrichtung derart ansteuert, dass eine vorbestimmte Menge an Säure in den Probenbehälter 1 eingeleitet wird (vgl. Figur 2). Nun wird die Probe über eine vorbestimmte Dauer gekocht. Dabei steuert die Steuereinheit die Heizung 30, so dass die gewünschte Temperatur eingehalten wird (vgl. Figur 3).

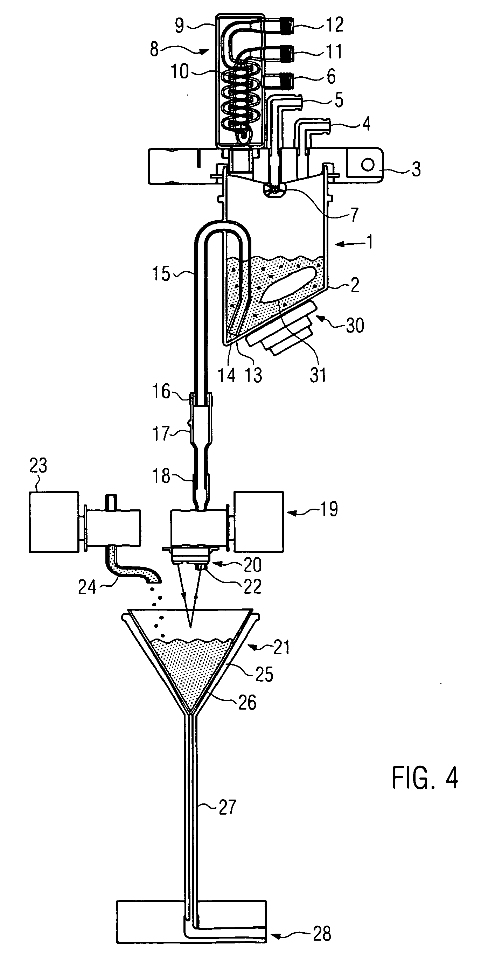

Nachfolgend wird durch die Steuereinheit die Benetzungseinrichtung 23 betätigt, so dass zunächst der Filtertrichter 25 mit der Benetzungsflüssigkeit gefüllt und das Filterpapier 26 durchtränkt wird. Die überschüssige Benetzungsflüssigkeit wird über das Ablaufrohr 27 in den Ablauf 28 abgeführt (vgl. Figur 4).

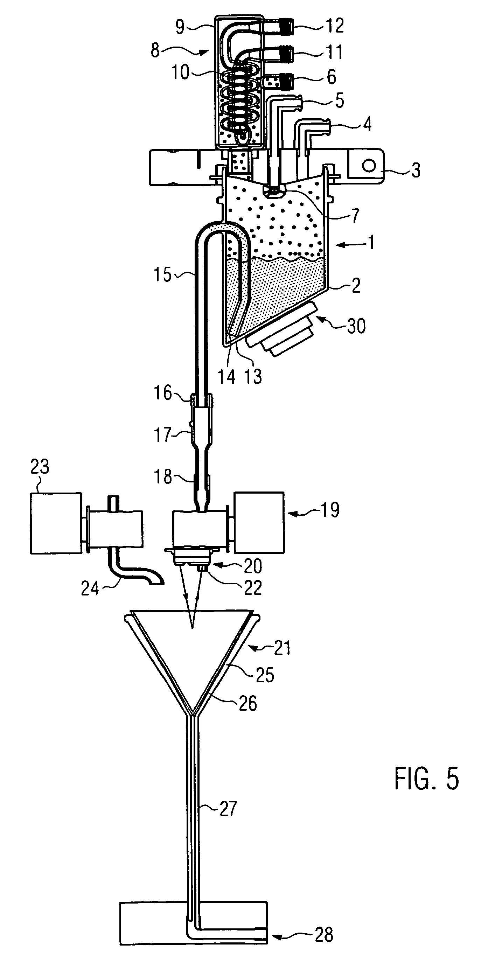

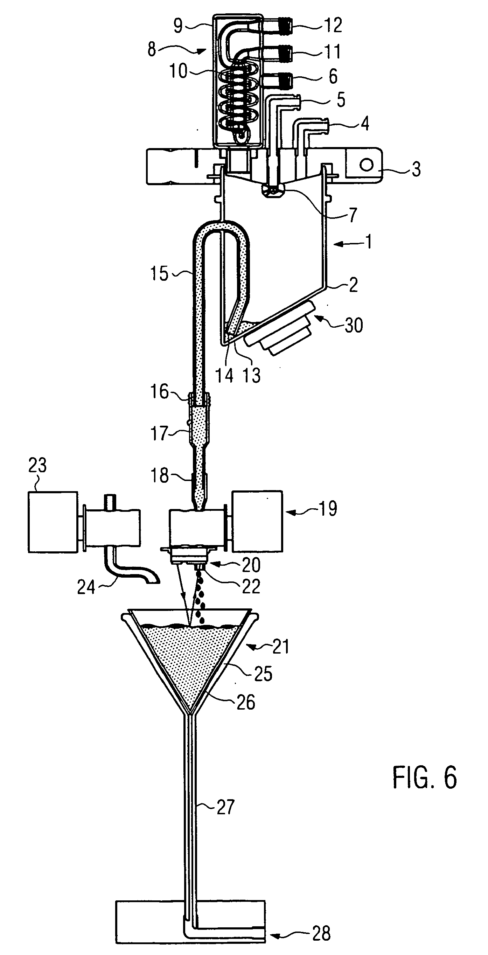

Danach wird das Druckluftventil geöffnet, so dass Druckluft in den Probenbehälter 1 eingeleitet und der Druck in dem Probenbehälter 1 erhöht wird. Hierdurch steigt die in dem Probenbehälter 1 befindliche Säure durch das behälterseitige Ende 14 des Siphons 15 in diesem auf, überwindet das Knie des Siphons 15 und fließt in Richtung auf das Ventil 19 (vgl. Figur 5). Sofern der Füllstand in dem Filter 21 einen vorbestimmten Wert unterschreitet, bleibt das Ventil 19 geöffnet. Dementsprechend fließt die aus dem Probenbehälter abgeleitete Säure zunächst in den Filter 21, dessen Füllstand ansteigt. Erreicht der Füllstand den vorbestimmten Wert, schließt der Sensor 22 das Ventil 19 (vgl. Figur 6). Noch nicht in den Filter 21 überführte Säure steht in dem Einlassstutzen 18 bzw. dem Siphon 15 an. Ein weiterer Rest der Säure kann sich in dem Probenbehälter 1 befinden. Nachdem der Füllstand aufgrund der Filtration und der Ableitung der Säure durch das Ablaufrohr 27 hinreichend abgesenkt worden ist, öffnet der Sensor 22 erneut das Ventil 19 und weitere Säure kann abfließen und gefiltert werden.

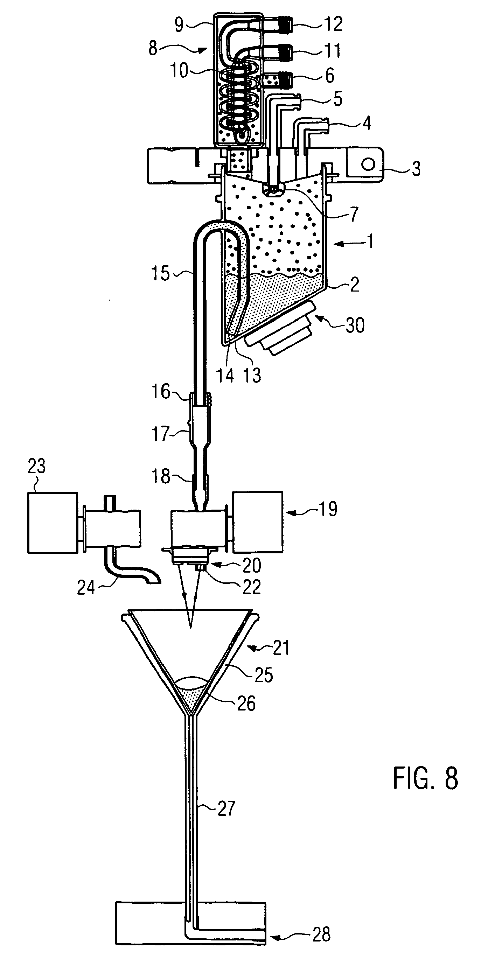

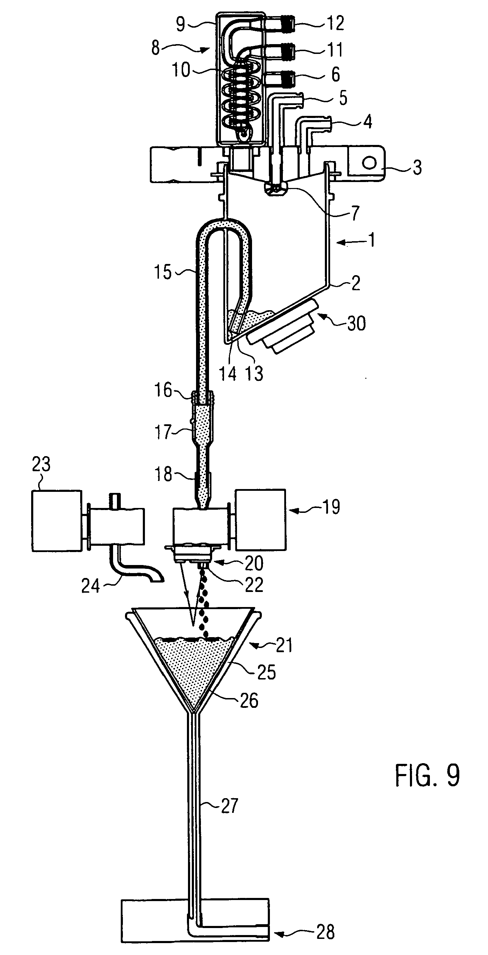

Nachdem im Wesentlichen die gesamte Säure aus dem Probenbehälter 1 entfernt worden ist beginnt die Spülphase, in der zunächst die Spülwasserdosiereinrichtung betätigt wird, um Spülwasser durch den Spülmittelanschluss 5 in den Probenbehälter 1 einzuleiten. Das Spülmittel, erwärmtes destilliertes Wasser passiert hierbei den Duschkopf 7 und verteilt sich entlang der Innenwandungen des Probenbehälters 1. Nachdem eine vorbestimmte Menge Spülmittel in den Probenbehälter 1 eingeleitet worden ist, wird vorzugsweise bei Einschalten der Heizung 30 der Probenbehälter gespült. Erneut wird das Druckventil geöffnet, um Druckluft in den Probenbehälter 1 einzuleiten (vgl. Figur 8). Das Spülwasser fließt in der vorstehend bereits in Bezug auf die Säure beschriebenen Weise durch den Siphon 15 ab und wird gesteuert durch das Ventil 19 in den Filter 21 eingeleitet (vgl. Figur 9).

Danach kann ein weiterer Spülgang folgen, bei dem erneut Spülwasser über den Spülwasseranschluss 5 in den Probenbehälter 1 eingeleitet wird. Dieser weitere Spülgang kann beispielsweise dadurch gesteuert werden, dass der Sensor 22 beim Ableiten des Spülwassers aus dem ersten Spülgang das Erreichen des maximalen Füllstandes an die Steuereinheit meldet, die daraufhin die Spülwasserdosiereinrichtung betätigt.

Die Anzahl der hintereinander folgenden Spülschritte, wie auch die Länge der Spülschritte kann über die Steuereinheit frei bestimmt werden. Ebenso ist es möglich, mehrere Aufschlussphasen hintereinander ablaufen zu lassen. Sowohl die Menge der in den Probenbehälter 1 eingeleiteten Säure, wie auch die Temperatur während der Aufschlussphase und die Menge und die Temperatur der Spülflüssigkeit können über die Steuereinheit frei gewählt werden.

Während der Aufschlussphase, d.h. während des Kochens der Probe 31, wie auch während der Spülphase bei eingeschalteter Heizung 30 verhindert der Kondensator 8 ein Verdampfen der in dem Probenbehälter 1 enthaltenen Flüssigkeit, welche an der Kühlmittelleitung 10 kondensiert und durch eine zwischen der Kondensatorsäule 9 und dem Probenaufnahmeraum gebildete Kondensatoröffnung 29 in den Probenbehälter 1 zurücktropft.

### Bezugszeichenliste

- 1: Probenbehälter
- 2: Probenglas
- 3: Deckel
- 4: Säureanschluss
- 5: Spülwasseranschluss
- 6: Druckluftanschluss
- 7: Duschkopf
- 8: Kondensator
- 9: Kondensatorsäule
- 10: Kühlmittelleitung
- 11: Kühlwassereinlass
- 12: Kühlwasserauslass
- 13: Probenbehälterboden
- 14: behälterseitiges Ende des Siffon
- 15: Siffon
- 16: anderes Ende des Siffon
- 17: Adapterstück
- 18: Einlassstutzen
- 19: Ventil
- 20: Füllstands-Sensoreinheit
- 21: Filter
- 22: Sensor
- 23: Benetzungseinrichtung
- 24: Rohrstück
- 25: Filtertrichter
- 26: Filterpapier
- 27: Ablaufrohr
- 28: Ablauf
- 29: Kondensatoröffnung
- 30: Heizung
- 31: Laborprobe

## Patentansprüche

1. Hydrolysevorrichtung zur diskontinuierlichen Hydrolyse von Laborproben (31) mit einem verschließbaren Probenbehälter (1), der mit einem Säure-, einem Spülwasser- und einem Gasanschluss (4; 5; 6) versehen ist und an dem ein bis an den Behälterboden (13) reichender Siffon (15) angeordnet ist.

2. Hydrolysevorrichtung nach Anspruch 1, **dadurch gekennzeichnet, dass** der Säure-, der Spülmittel- und der Gasanschluss (4; 5; 6) an einem Deckel (3) des Probenbehälters (1) angeordnet sind.

3. Hydrolysevorrichtung nach Anspruch 1 oder 2, **dadurch gekennzeichnet, dass** ein mit dem Probenbehälter (1) kommunizierender Kondensator (8) vorgesehen ist.

4. Hydrolysevorrichtung nach Anspruch 3, **dadurch gekennzeichnet, dass** der Kondensator (8) an dem Deckel (3) montiert ist und diesen überragt.

5. Hydrolysevorrichtung nach Anspruch 3 oder 4, **dadurch gekennzeichnet, dass** der Kondensator (8) eine an dem Deckel (3) befestigte und über eine Kondensatoröffnung (29) mit dem Probenbehälter (1) kommunizierende Kondensatorsäule (9) umfasst, die eine Kühlmittelleitung (10) umgibt.

6. Hydrolysevorrichtung nach einem der vorherigen Ansprüche, **dadurch gekennzeichnet, dass** der Spülmittelanschluss (5) behälterseitig in einen Sprühkopf (7) mündet.

7. Hydrolysevorrichtung nach einem der vorherigen Ansprüche, **gekennzeichnet durch** eine dem Behälterboden (13) des Probenbehälters (1) zugeordnete Heizeinrichtung (30).

8. Hydrolysevorrichtung nach einem der vorherigen Ansprüche, **dadurch gekennzeichnet, dass** der Behälterboden (13) eine Senke aufweist, in der das Ende (14) des Siffon (15) angeordnet ist.

9. Hydrolysevorrichtung nach einem der vorherigen Ansprüche, **dadurch gekennzeichnet, dass** das andere Ende (16) des Siphon (15) an einem Ventil (19) angeschlossen ist, das von einem die Füllhöhe einer nachgeordneten Filtriereinrichtung (21) erfassenden Sensor (22) gesteuert wird.

10. Hydrolysevorrichtung nach Anspruch 9, **dadurch gekennzeichnet, dass** der Sensor (22) ein auf den Flüssigkeitsspiegel der Filtriereinrichtung (21) gerichteter und durch Reflektion die Füllhöhe derselben ermittelnder optischer Sensor (22) ist.

11. Hydrolysevorrichtung nach einem der vorherigen Ansprüche, **gekennzeichnet durch** eine mit dem Säureanschluss (4) verbundenen Säuredosiereinrichtung, ein mit dem Gasanschluss (6) verbundenes Druckluftventil und eine mit dem Spülmittelanschluss (5) verbundenen Spülwasserdosiereinrichtung sowie einer den Säuredosiereinrichtung, das Druckluftventil und der Spülwasserdosiereinrichtung steuernden Steuereinheit.

12. Hydrolysevorrichtung nach Anspruch 11, **gekennzeichnet durch** eine die Filtriereinrichtung (21) benetzende und von der Steuereinheit gesteuerte Benetzungseinrichtung (23).

13. Verfahren zur diskontinuierlichen Hydrolyse einer Laborprobe (31), bei dem die Laborprobe (31) in einen Probenbehälter (1) eingebracht, in Säure gekocht und nachfolgend gespült wird, **gekennzeichnet durch** folgende Schritte:
Einbringen der Probe (31) in einen verschließbaren Probenbehälter (1) und dichtes Verschließen des Probenbehälters (1);
Starten eines automatisiert ablaufenden Hydrolyseprogramms, welches
a) eine Säuredosiereinrichtung derart steuert, dass eine vorbestimmte Säuremenge in den Probenbehälter (1) eingebracht wird;
b) eine dem Probenbehälter (1) zugeordnete Heizeinrichtung derart steuert, dass die in dem Probenbehälter (1) befindliche Säure über eine vorbestimmte Kochdauer auf einer vorbestimmte Temperatur gehalten wird;
c) sodann ein dem Probenbehälter (1) zugeordnetes Druckventil ansteuert, um den Innendruck in dem Probenbehälter (1) so weit zu erhöhen, dass die Säure **durch** einen Siphon (15) des Probenbehälters (1) aufsteigt und aus dem Probenbehälter (1) abfließt und in einer nachgeordneten Filtriereinrichtung (21) gefiltert wird;
d) danach eine mit dem Probenbehälter (1) kommunizierende Spülmitteldosiereinrichtung derart ansteuert, dass ein vorbestimmbares Volumen an Spülflüssigkeit in den Probenbehälter (1) eingebracht wird und
e) nach Ablauf einer vorbestimmbaren Spüldauer schließlich erneut das Druckventil ansteuert, um den Innendruck in dem Probenbehälter (1) so weit zu erhöhen, dass die Spülflüssigkeit **durch** den Siffon (15) aufsteigt und aus dem Probenbehälter (1) abfließt und ebenfalls gefiltert wird.

14. Verfahren nach Anspruch 13, **dadurch gekennzeichnet,**
**dass** das Druckventil in den Schritten c) und e) abhängig von dem Füllstand der Filtriereinrichtung (21) gesteuert wird.

15. Verfahren nach Anspruch 13 oder 14, **dadurch gekennzeichnet,**
**dass** das die Spülflüssigkeit von dem Einbringen in den Probenbehälter (1) erwärmt wird.

16. Verfahren nach wenigstens einem der Ansprüche 13 bis 15, **dadurch gekennzeichnet, dass** vor dem Schritt c) ein in die Filtriereinrichtung eingelegtes Filterpapier (26) benetzt wird.

17. Verfahren nach wenigstens einem der Ansprüche 13 bis 16, **dadurch gekennzeichnet, dass** die Schritte a), b) und c) und/oder die d) und e) mehrmals nacheinander wiederholt werden.

## Claims

1. Hydrolysis apparatus for discontinuous hydrolysis of laboratory samples (31) having a closable sample container (1) which is provided with an acid connection, a washing water connection and a gas connection (4; 5; 6) and on which a siphon (15) reaching as far as the container floor (13) is disposed.

2. Hydrolysis apparatus as claimed in claim 1, **characterised in that** the acid, washing agent and gas connections (4; 5; 6) are disposed on a cover (3) of the sample container (1).

3. Hydrolysis apparatus as claimed in claim 1 or 2, **characterised in that** a condenser (8) communicating with the sample container (1) is provided.

4. Hydrolysis apparatus as claimed in claim 3, **characterised in that** the condenser (8) is mounted on the cover (3) and protrudes beyond it

5. Hydrolysis apparatus as claimed in claim 3 or 4, **characterised in that** the condenser (8) has a condenser column (9) which is attached to the cover (3), communicates with the sample container (1) via a condenser orifice (29) and surrounds a coolant line (10).

6. Hydrolysis apparatus as claimed in any one of the preceding claims, **characterised in that** the washing agent connection (5) issues on the container side into a spray head (7).

7. Hydrolysis apparatus as claimed in any one of the preceding claims, **characterised by** a heating device (30) allocated to the container floor (13) of the sample container (1).

8. Hydrolysis apparatus as claimed in any one of the preceding claims, **characterised in that** the container floor (13) has a depression in which the end (14) of the siphon (15) is disposed.

9. Hydrolysis apparatus as claimed in any one of the preceding claims, **characterised in that** the other end (16) of the siphon (15) is connected to a valve (19) which is controlled by a sensor (22) detecting the filling level of a filtering device (21) disposed downstream.

10. Hydrolysis apparatus as claimed in claim 9, **characterised in that** the sensor (22) is an optical sensor (22) arranged with respect to the liquid level of the filtering device (21) and, by means of reflection, determining the filling level thereof.

11. Hydrolysis apparatus as claimed in any one of the preceding claims, **characterised by** an acid metering device connected to the acid connection (4), a compressed air valve connected to the gas connection (6) and a washing water metering device connected to the washing agent connection (5), and by a control unit controlling the acid metering device, the compressed air valve and the washing water metering device.

12. Hydrolysis apparatus as claimed in claim 11, **characterised by** a wetting device (23) wetting the filtering device (21) and controlled by the control unit.

13. Method for discontinuous hydrolysis of a laboratory sample (31), wherein the laboratory sample (31) is introduced into a sample container (1), boiled in acid and then washed, **characterised by** the following steps:
introducing the sample (31) into a closable sample container (1) and closing the sample container (1) in a sealed manner;
starting an automated hydrolysis program which
a) controls an acid metering device in such a way that a predetermined quantity of acid is introduced into the sample container (1);
b) controls a heating device allocated to the sample container (1), in such a way that the acid located in the sample container (1) is maintained at a predetermined temperature for a predetermined boiling time;
c) then actuates a pressure valve allocated to the sample container (1) in order to raise the internal pressure in the sample container (1) to such an extent that the acid rises through a siphon (15) in the sample container (1) and flows out of the sample container (1) and is filtered in a filtering device (21) disposed downstream;
d) then actuates a washing agent metering device communicating with the sample container (1), in such a way that a predeterminable volume of washing liquid is introduced into the sample container (1) and
e) finally, after a predeterminable washing time is over, actuates the pressure valve again in order to raise the internal pressure in the sample container (1) to such an extent that the washing liquid rises through the siphon (15) and flows out of the sample container (1) and is also filtered.

14. Method as claimed in claim 13, **characterised in that** the pressure valve is controlled in steps c) and e) in dependence upon the filling level of the filtering device (21).

15. Method as claimed in claim 13 or 14, **characterised in that** the washing liquid is heated by being introduced into the sample container (1).

16. Method as claimed in at least one of claims 13 to 15, **characterised in that** before step c) a filter paper (26) inserted into the filtering device is wetted.

17. Method as claimed in at least one of claims 13 to 16, **characterised in that** the steps a), b) and c) and/or steps d) and e) are repeated a number of times in succession.

## Revendications

1. Dispositif d'hydrolyse pour l'hydrolyse en discontinu d'échantillons de laboratoire (31), comprenant un récipient pour échantillons (1) apte à être fermé, qui est muni d'un raccord pour les acides, d'un raccord pour l'eau de rinçage et d'un raccord pour les gaz (4 ; 5 ; 6) et contre lequel est disposé un siphon (15) qui s'étend jusqu'au fond (13) du récipient.

2. Dispositif d'hydrolyse selon la revendication 1, **caractérisé en ce que** le raccord pour les acides, le raccord pour les agents de rinçage et le raccord pour les gaz (4 ; 5 ; 6) sont disposés contre un couvercle (3) du récipient pour échantillons (1).

3. Dispositif d'hydrolyse selon la revendication 1 ou 2, **caractérisé en ce qu'**il est muni d'un condenseur (8) communiquant avec le récipient pour échantillons (1).

4. Dispositif d'hydrolyse selon la revendication 3, **caractérisé en ce que** le condenseur (8) est monté contre le couvercle (3) et fait saillie par rapport à ce dernier.

5. Dispositif d'hydrolyse selon la revendication 3 ou 4, **caractérisé en ce que** le condenseur (8) comprend une colonne de condensation fixée au couvercle (3) et communiquant, via une ouverture de condenseur (29), avec le récipient pour échantillons (1), la colonne entourant un conduit (10) pour réfrigérant.

6. Dispositif d'hydrolyse selon l'une quelconque des revendications précédentes, **caractérisé en ce que** le raccord (5) pour les agents de rinçage débouche, coté récipient, dans une tête de pulvérisation (7).

7. Dispositif d'hydrolyse selon l'une quelconque des revendications précédentes, **caractérisé par** un mécanisme de chauffage (30) attribué au fond (13) du récipient pour échantillons (1).

8. Dispositif d'hydrolyse selon l'une quelconque des revendications précédentes, **caractérisé en ce que** le fonds (13) du récipient présente une cuvette dans laquelle est disposée l'extrémité (14) du siphon (15).

9. Dispositif d'hydrolyse selon l'une quelconque des revendications précédentes, **caractérisé en ce que** l'autre extrémité (16) du siphon (15) est raccordée à une valve (19) qui est commandée par un capteur (22) qui enregistre la hauteur de remplissage d'un dispositif de filtration (21) monté à la suite.

10. Dispositif d'hydrolyse selon la revendication 9, **caractérisé en ce que** le capteur (22) est un capteur (22) orienté dans la direction du niveau de liquide du dispositif de filtration (21) et qui détermine par réflexion la hauteur de remplissage dudit mécanisme.

11. Dispositif d'hydrolyse selon l'une quelconque des revendications précédentes, **caractérisé par** un mécanisme de dosage d'acides relié au raccord (4) pour les acides, par une valve pneumatique reliée au raccord (6) pour les gaz et par un mécanisme de dosage des eaux de rinçage relié au raccord (5) pour les eaux de rinçage, ainsi que par une unité de commande qui commande le mécanisme de dosage des acides, la valve pneumatique et le mécanisme de dosage des eaux de rinçage.

12. Dispositif d'hydrolyse selon la revendication 11, **caractérisé par** un mécanisme de mouillage (23) qui mouille le dispositif de filtration (21) et qui est commandé par l'unité de commande.

13. Procédé pour l'hydrolyse en discontinu d'un échantillon de laboratoire (31), dans lequel l'échantillon de laboratoire (31) est introduit dans un récipient pour échantillons (1), est porté à ébullition dans un acide et est ensuite rincé, **caractérisé par** les étapes suivantes consistant à :
introduire l'échantillon (31) dans un récipient pour échantillons (1) apte à être fermé et fermer hermétiquement le récipient pour échantillons (1) ;
lancer un programme d'hydrolyse se déroulant de manière automatique qui
a) commande un mécanisme de dosage d'acide de telle sorte qu'une quantité d'acide prédéfinie est introduite dans le récipient pour échantillons (1) ;
b) commande un mécanisme de chauffage attribué au récipient pour échantillons (1) de telle sorte que l'acide se trouvant dans le récipient pour échantillons (1) est maintenu à une température prédéfinie pendant une durée de cuisson prédéfinie ;
c) commande ensuite une valve pneumatique attribuée au récipient pour échantillons (1) afin d'élever la pression interne régnant dans le récipient pour échantillons (1) à un point tel que l'acide monte à travers un siphon (15) du récipient pour échantillons (1) et s'écoule hors du récipient pour échantillons (1) et est filtré dans un dispositif de filtration (21) monté à la suite ;
d) commande ensuite un mécanisme de dosage d'agent de rinçage communiquant avec le récipient pour échantillons (1) de façon à introduire un volume prédéfinissable de liquide de rinçage dans le récipient pour échantillons (1) ; et
e) enfin, après le déroulement d'un laps de temps de rinçage prédéfinissable, commande une nouvelle fois la valve pneumatique pour élever la pression interne régnant dans le récipient pour échantillons (1) à un point tel que le liquide de rinçage monte à travers le siphon (15) et s'écoule hors du récipient pour échantillons (1) et est également filtré.

14. Procédé selon la revendication 13, **caractérisé en ce que** la valve pneumatique est commandée, dans les étapes c) et e) indépendamment du niveau de remplissage du dispositif de filtration (21).

15. Procédé selon la revendication 13 ou 14, **caractérisé en ce que** le liquide de rinçage est chauffé avant son introduction dans le récipient pour échantillons (1).

16. Procédé selon au moins une des revendications 13 à 15, **caractérisé en ce que,** avant l'étape c), on mouille un papier filtrant (26) inséré dans le dispositif de filtration.

17. Procédé selon au moins une des revendications 13 à 16, **caractérisé en ce qu'**on répète plusieurs fois successivement les étapes a) b) et c) et/ou les étapes d) et e).
